(19) Europäisches Patentamt
European Patent Office
Office européen des brevets

(11) EP 1 609 418 A1

(12) EUROPEAN PATENT APPLICATION

(43) Date of publication:
28.12.2005 Bulletin 2005/52

(51) Int Cl.⁷: **A61B 5/16**

(21) Application number: 05253655.4

(22) Date of filing: 14.06.2005

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: 22.06.2004 JP 2004183284

(71) Applicant: **SONY CORPORATION**
**Tokyo 141-0001 (JP)**

(72) Inventors:
 • **Shirai, Katsuya, c/o Sony Corporation**
  **Tokyo 141-0001 (JP)**
 • **Sako, Yoichiro, c/o Sony Corporation**
  **Tokyo 141-0001 (JP)**
 • **Terauchi, Toshiro, c/o Sony Corporation**
  **Tokyo 141-0001 (JP)**

 • **Inoue, Makoto, c/o Sony Corporation**
  **Tokyo 141-0001 (JP)**
 • **Asukai, Masamichi, c/o Sony Corporation**
  **Tokyo 141-0001 (JP)**
 • **Miyajima, Yasushi, c/o Sony Corporation**
  **Tokyo 141-0001 (JP)**
 • **Makino, Kenichi, c/o Sony Corporation**
  **Tokyo 141-0001 (JP)**
 • **Takai, Motoyuki, c/o Sony Corporation**
  **Tokyo 141-0001 (JP)**

(74) Representative: **Merryweather, Colin Henry**
 **J.A. Kemp & Co.**
 **14 South Square**
 **Gray's Inn**
 **London WC1R 5JJ (GB)**

(54) **Apparatus for estimating the psychological state of a subject and video/sound reproduction apparatus**

(57) A bio-information processing apparatus includes a plurality of bio-information sensors for obtaining a plurality of measured bio-information values of a subject and outputting the plurality of measured bio-information values as a plurality of biological signals, and a circuit for estimating the psychological state and intensity of the psychological state of a subject from the plurality of biological signals and from one of initial bio-information values and reference bio-information values.

FIG. 1

**Description**

The present invention contains subject matter related to Japanese Patent Application JP 2004-183284 filed in the Japanese Patent Office on June 22, 2004, the entire contents of which are incorporated herein by reference.

The present invention relates to a bio-information processing apparatus and a video/sound reproduction apparatus.

Recently, attempts have been made to infer a person's psychology from the person's bio-information and utilize this psychological data in biofeedback and user interfaces.

For example, there is a method of inferring a person's psychology from the person's facial expressions. In this method, the facial expression of a subject is captured by a video camera. Then, the facial expression is compared with expressional patterns and movement patterns of the facial muscles stored in a database in advance. In this way, the facial expression can be categorized into different psychological states of laughter, anger, grief, confusion, and astonishment (for example, refer to Japanese Unexamined Patent Application Publication Nos. 3-252775 and 2000-76421).

There is also a method of inferring a person's psychology from a fluctuation of the person's pulse rate (or heart beat rate). In this method, the subject wears an electrocardiograph or a pulse sensor to measure his or her pulse rate. By observing the fluctuation in the subject's pulse rate, the subject's tension or emotional change can be detected (for example, refer to Japanese Patent Unexamined Patent Application Publication Nos. 7-323162 and 2002-23918).

There is also a method of inferring a person's psychology from a plurality of biological signals of, for example, optical blood flow, electrocardiographic activity, electrodermal activity, and skin temperature. When employing such a method, the subject wears a watch-type sensor to optically measure blood flow, electrocardiographic activity, electrodermal activity, and skin temperature.

Then, from the measurements, a characteristic vector extracting the characteristics of each index is generated. The characteristic vector is compared with a plurality of emotional state values stored in a database in advance. In this way, the subject's psychology can be categorized into different psychological states, such as joy, relief, satisfaction, calmness, overconfidence, grief, dissatisfaction, anger, astonishment, fear, depression, and stress (for example, refer to Japanese Patent Unexamined Patent Application Publication No. 2002-112969).

If the subject's psychological state can be inferred from such measurements, for example, if an operator of a device suffers a disability that makes it difficult for him or her to operate the device, an operation environment most desirable for the operator's psychological state can be provided automatically.

However, it is often difficult to infer one's psychology by employing the above-described methods. For example, there are facial expressions, such as 'astonishment' and 'confusion,' that are difficult to distinguish from each other. Furthermore, it is known that one's pulse rate shows the same kind of change when the level of arousal is high while the level of valence is either positively high (i.e., when the subject is feeling pleasure) or negatively high (i.e., when the subject is feeling displeasure). For this reason, valence inferred from pulse rate when arousal is high may be incorrect.

The main object of the above-described methods is to merely categorize one's psychology from bio-information. Therefore, the intensity of one's psychological state, such as "extreme pleasure" or "moderate pleasure," cannot be measured correctly.

The apparatuses according to embodiments of the present invention combine a plurality of bio-information items to infer a subject's psychological state and the intensity of the psychological state. Moreover, according to the psychological state of the subject, the apparatuses provide an environment, including images and sounds, optimal to the subject's psychology.

A video/sound reproduction apparatus according to an embodiment of the present invention includes a reproduction unit for reproducing at least one of an image signal and a sound signal, a plurality of bio-information sensors for obtaining a plurality of measured bio-information values of a subject and outputting the plurality of measured bio-information values as a plurality of biological signals, a circuit for estimating the psychological state and intensity of the psychological state of the subject from the plurality of biological signals and from one of initial bio-information values and reference bio-information values, and a modification unit for modifying at least one of the image signal and the sound signal reproduced by the reproduction unit in accordance with the results estimated by the circuit.

In this way, the video/sound reproduction apparatus is capable of inferring a subject's psychological state and the intensity of the psychological state by using a plurality of bio-information values collected by a plurality of bio-information sensors to obtain the values of arousal and valence of the user. Then, images and sound can be reproduced in accordance with the obtained results such that the user's psychological state is maintained at an optimal state.

To allow better understanding an embodiment of the present invention will now be described by way of non-limitative example with reference to the accompanying drawings in which:

Fig. 1 is a schematic diagram of a video/sound reproduction apparatus according to an embodiment of the present invention;

Fig. 2 illustrates output data from a bio-information sensor employed in an embodiment of the present invention;

Fig. 3 illustrates the use of the bio-information sensor employed in an embodiment of the present invention;

Fig. 4 is a flow chart showing a control flow according to an embodiment of the present invention;

Fig. 5 illustrates a graph representing an embodiment of the present invention; and

Fig. 6 illustrates another graph representing an embodiment of the present invention.

According to an embodiment of the present invention, the biological state of a subject is measured by various bio-information sensors. From biological signals sent from the various bio-information sensors, values of arousal and valence, which are indices representing the subject's psychological state, are obtained. In accordance with the values of arousal and valence, the subject's environment can be changed.

[1] Video/sound Reproduction Apparatus

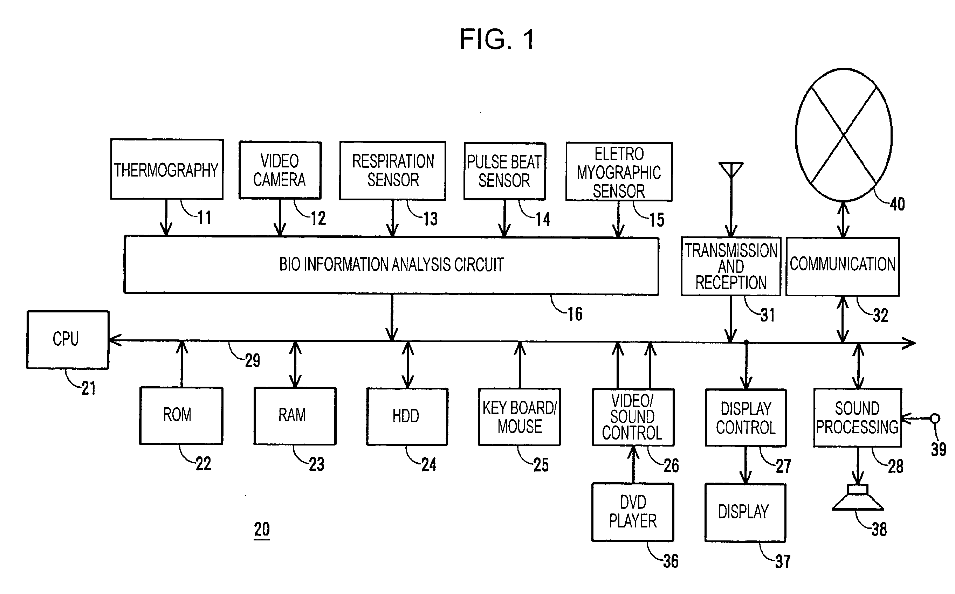

Fig. 1 illustrates a video/sound reproduction apparatus according to an embodiment of the present invention. Images and sounds reproduced by the video/sound reproduction apparatus are controlled accordingly to the values of arousal and valence of the subject.

In order to realize such control the video/sound reproduction apparatus includes a thermograph 11 and a video camera 12 as noncontact bio-information sensors for collecting bio-information from a user without making physical contact. The outputs from the thermograph 11 and the video camera 12 are sent to a bio-information analysis circuit 16.

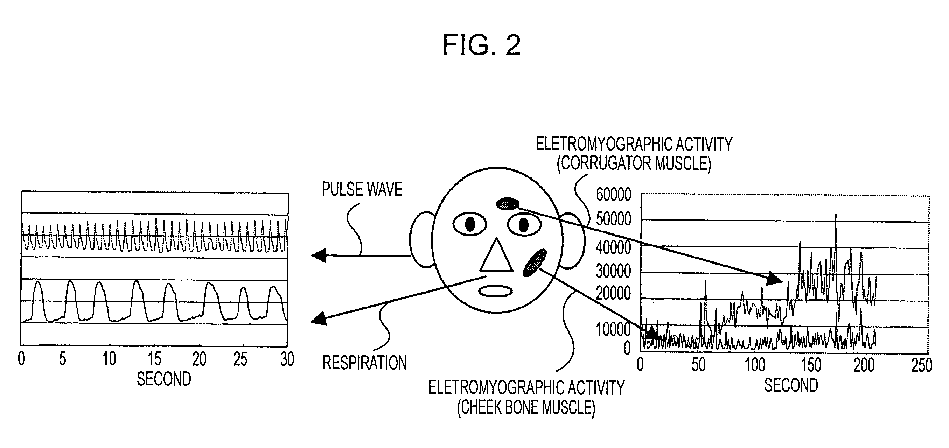

In such case, as illustrated in Fig. 2, the surface temperature of the user's face is measured using the thermograph 11. The measurement results of the thermograph 11 are analyzed by the bio-information analysis circuit 16. Through this analysis, the respiration rate and pulse rate of the user is determined indirectly from the change in temperatures of the user's nostrils and the surrounding area over time. At the same time, the user's face is captured by the video camera 12. The captured image of the user's face is sent to the bio-information analysis circuit 16 to determine the displacement of predetermined points on the face, such as points on the cheek and forehead, and between the eyebrows. More specifically, when the cheek bone muscle and the corrugator muscle expand or contract, the predetermined points are displaced. The amount of expansion or contraction of the muscles can be determined from the displacement of the predetermined points. As a result, electromyographic activity can be measured.

The video/sound reproduction apparatus according to an embodiment includes a respiration sensor 13, a pulse sensor 14, and an electromyographic sensor 15 as contact bio-information sensors worn by the user to collect bio-information of the user. The outputs of these bio-information sensors are also sent to the bio-information analysis circuit 16.

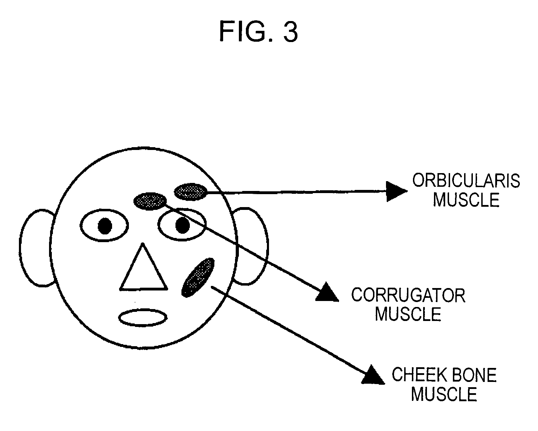

In this case, the respiration sensor 13 is attached to the user's chest or abdominal area and the pulse sensor 14 is attached to the user's finger tip. The outputs of the respiration sensor 13 and the pulse sensor 14 are sent to the bio-information analysis circuit 16 so that the change in the user's respiration and pulse is determined. The electromyographic sensor 15, as illustrated in Fig. 3, has electrodes attached to the user's cheek, forehead and area between the eyebrows. The output from the electromyographic sensor 15 is sent to the bio information analysis circuit 16 so that the active parts of the user's face and the magnitude and change of fluctuations of the electromyographic activity is determined based on the output.

The thermograph 11, video camera 12, respiration sensor 13, pulse sensor 14, electromyographic sensor 15, and the outputs from all of these sensors do not have to be used: only the sensors suitable for conditions such as the user's listening conditions and measurement conditions may be selected for use.

The analytic results of the bio-information analysis circuit 16 are sent to a microcomputer 20, and the arousal and valence of the user are computed. In accordance with the obtained results, desirable video image and sound are reproduced. More specifically, the microcomputer 20 includes a central processing unit (CPU) 21, a read only memory (ROM) 22 storing various programs, and a random access memory (RAM) 23 used as a work area, wherein each of the units are mutually connected via a system bus 29.

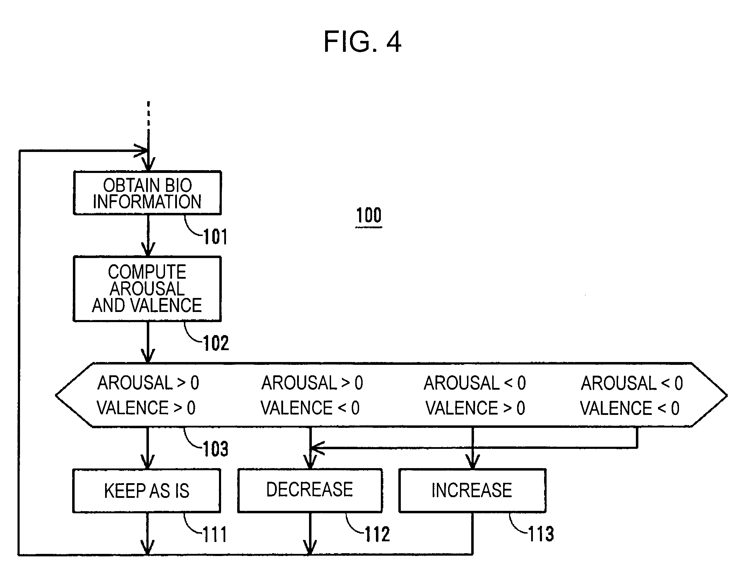

In this case, the ROM 22 stores, for example, a routine 100, as illustrated in Fig. 4, as part of a program executed by the CPU 21. Details of the routine 100 will be described below. The routine 100 is configured to control an image signal or a sound signal in accordance with the user's bio-information such that video image and sound can be perceived by the user with pleasure. As illustrated in Fig. 4, the routine 100 according to an embodiment is part of a program, and this part includes only the processes that are included in the scope of the present invention.

The microcomputer 20 includes a hard disk drive 24 used as a mass storage device and a user interface 25, such as a keyboard or a mouse. Both the hard disk drive 24 and the user interface 25 are also connected to the system bus 29. According to this embodiment, a digital versatile disk (DVD) player 36 is provided as a source of image signals and sound signals. The DVD player 36 is connected to the system bus 29 via a video/sound control circuit 26.

In this case, the video/sound control circuit 26 is capable of controlling the image signal reproduced by the DVD player 36 to modify the conditions, such as contrast, brightness, hue, and saturation of color of a displayed image and controlling the reproduction speed of the DVD player 36. Furthermore, the video/sound control circuit 26 controls the sound signal reproduced by the DVD player 36 to control the volume, frequency characteristics, and reverberation of the reproduced sound.

The system bus 29 is connected to a display 37 via a display control circuit 27. An image signal output from the video/sound control circuit 26 is converted into a display signal by the display control circuit 27. This display signal is supplied to the display 37. A sound processing circuit 28 is connected to the system bus 29 to supply a sound signal to a speaker 38 via the sound processing circuit 28 and to supply a sound signal from a microphone 39 to the microcomputer 20 via the sound processing circuit 28.

Bio-information and other data of the user collected by the video/sound reproduction apparatus and other apparatuses may be transmitted between each apparatus by connecting the system bus 29 to a transmission and reception circuit 31 and a communication circuit 32. The communication circuit 32 is connected to other networks, such as the Internet 40.

According to the above-described structure, an image signal and a sound signal are reproduced by the DVD player 36 by operating the user interface 25. The image signal is supplied to the display 37 via the video/sound control circuit 26 and the display control circuit 27 so as to display an image on the display 37. Similarly, the sound signal is supplied to the speaker 38 via the video/sound control circuit 26 and the sound processing circuit 28 to play sound from the speaker 38.

At this time, the CPU 21 executes the routine 100 to compute the user's arousal and valence in response to the image displayed on the display 37 and the sound played from the speaker 38. Based on the computed values, the image and sound are controlled so that they are perceived by the user with pleasure.

More specifically, when the routine 100 is executed, first in Step 101, bio-information collected by the thermograph 11, video camera 12, respiration sensor 13, pulse sensor 14, and electromyographic sensor 15 is sent to the microcomputer 20 via the bio-information analysis circuit 16. Then, in Step 102, arousal and valence are computed based on the bio-information sent to the bio-information analysis circuit 16 in Step 101. The computation method will be described below. Both arousal and valence are obtained by computation in analog values that may be either positive or negative values.

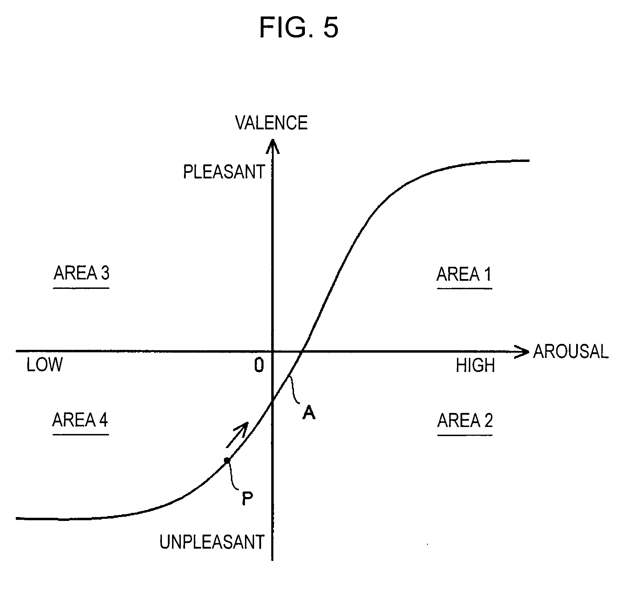

Subsequently, the process proceeds to Step 103. In Step 103, the signs (positive or negative) of the value of arousal and valence obtained in Step 102 are determined. Then, the next step in the process is determined in accordance with the combination of the signs of the values. In other words, since both arousal and valence may be either a positive value or a negative value, when arousal and valence are plotted on two-dimensional coordinate axes, the graph illustrated in Fig. 5 is obtained. According to this graph:

in Area 1, arousal>0 and valence>0 (arousal is high and the user is in a state of pleasure);
in Area 2, arousal>0 and valence<0 (arousal is high and the user is in a state of displeasure);
in Area 3, arousal<0 and valence>0 (arousal is low and the user is in a state of pleasure); and
in Area 4, arousal<0 and valence<0 (arousal is low and the user is in state of displeasure).

When the values of arousal and valence fall into Area 1, it is assumed that the user is perceiving the image and sound pleasantly, and the process proceeds from Step 103 to Step 111. In Step 111, the image signal and the sound signal supplied to the display 37 and the speaker 38, respectively, are not modified, and then the process proceeds to Step 101. In other words, when the values of arousal and valence fall into Area 1, it is inferred that the user is satisfied with the image and sound and thus the reproduction conditions of the image and sound are not changed.

However, when the values of arousal and valence fall into Area 2, it is assumed that the user is perceiving the image and sound with displeasure, and the process proceeds from Step 103 to Step 112. In Step 112, to remove the user's displeasure, for example, the level of the direct current and/or alternate current of the image signal sent to the display 37 is lowered to lower the brightness and/or contrast of the image displayed on the display 37. Similarly, for example, the level of the sound signal sent to the speaker 38 is lowered and/or the frequency characteristics of the sound signal are modified to lower the volume of the sound output from the speaker 38, weaken the low and high frequency bands of the sound signal, and/or weaken the rhythm of the sound. Then, the process proceeds to Step 101.

If the condition set in Step 112 continues for a predetermined period of time, this means the values of arousal and valence are not being improved and the user is still experiencing displeasure. In such a case, for example, the reproduction of image and sound can be terminated in Step 112.

When the values of arousal and valence fall into Area 3, the process proceeds from Step 103 to Step 113. In Step 113, contrary to Step 112, the user's degree of pleasure can be increased and/or feelings can be elevated, for example, by increasing the level of the direct current and/or alternating current of the image signal sent to the display 37 to increase the brightness and/or contrast of the image displayed on the display 37. Similarly, for example, the level of the sound signal sent to the speaker 38 can be increased and/or the frequency characteristics of the sound signal can be modified to increase the volume of the sound output from the speaker 38, strengthen the low and high frequency bands of the sound signal, and/or emphasize the rhythm of the sound. Then, the process proceeds to Step 101.

For example, if the user sets the video/sound reproduction apparatus to 'sleeping mode' using the user interface 25, images and sound can be reproduced so that the values of arousal and valence stay in Area 3 since images and sounds in this area will not interfere with the user's sleep.

When the values of arousal and valence fall into Area 4, it is assumed that the user is perceiving the image and sound with displeasure, and the process proceeds from Step 103 to Step 112. The user's displeasure is removed in the same manner as in the case in which the value of arousal and valence fall into Area 2.

Accordingly, by executing the routine 100, image and sound can be reproduced in a manner such that the user will always perceives the image and sound with pleasure.

In this way, the above-described video/sound reproduction apparatus is capable of inferring a user's psychological state and the intensity of the psychological state by using a plurality of bio-information values collected by a plurality of bio-information sensors (thermograph 11, video camera 12, respiration sensor 13, pulse sensor 14, and electromyographic sensor 15) to obtain the values of arousal and valence of the user. Then, images and sound can be reproduced in accordance with the obtained results such that the user's psychological state is maintained at an optimal state.

[2] Computing Arousal and Valence

In which area in the graph, illustrated in Fig. 5, the values of arousal and valence of the user falls can be determined by the processes described below in sections [2-1] and [2-2]. If, for example, the present values of arousal and valence of the user are at a point P, in Fig. 5, it can be determined in which direction along the curved line A including the point P the values of arousal and valence will change based on previous change history of the values.

Accordingly, the best image and sound for the user's psychological state can always be provided. Moreover, if the user is in a positive psychological state, this positive state can be maintained and if the user is in a negative psychological state, this state can be improved.

[2-1] Computing Arousal

Arousal can be determined from the deviation of the measured respiratory rate and pulse rate of the user from initial or standard values. The bio-information sensors used to measure the user's respiratory rate and pulse rate may be either noncontact-type sensors or contact-type sensors. Arousal can be computed using the formulas below:

$$\text{Arousal} = R_{rm} - R_{rr} \tag{1}$$

where, $R_{rm}$ represents the measured respiration rate per unit time and $R_{rr}$ represent the initial or standard respiration rate per unit time, or

$$\text{Arousal} = P_{rm} - P_{rr} \tag{2}$$

where, $P_{rm}$ represents the measured pulse rate per unit time and $P_{rr}$ represent the initial or standard pulse rate per unit time. Formula (2) may be used to compute arousal even when the heart rate is being used as pulse rate.

[2-2] Computing Valence

Valence can be determined by applying the output value of the electromyographic sensor 15 to, for example, Formula (3) described below:

$$\text{Valence} = \int |V_{emg}(t)| dt - V_{emg\_init} \tag{3}$$

where $V_{emg}$ represents the magnitude of the fluctuation of the measured value of electromyographic activity and $V_{emg\_init}$ represents the integrated value (initial value) of the magnitude of fluctuation of electromyographic activity, or

$$\text{Valence}=\int |V_{emg}(t)| dt - V_{emg\_ref} \tag{4}$$

where $V_{emg\_ref}$ represents the magnitude of the fluctuation of the integrated value (reference value) of electromyographic activity.

The positive value of valence is determined based on the electromyographic measurements taken from the cheek bone muscle and the negative value of valence is determined based on the electromyographic measurements taken from the corrugator muscle or the orbicularis muscle.

When measurements are taken using a noncontact sensor, as illustrated in Fig. 3, such electromyographic activity will have to be measured indirectly. In such a case, electromyographic activity can be measured by measuring the displacement of a predetermined point on the user's face or the change in the distance between points on the user's face.

In other words, force f(r) of a two-dimensional harmonic vibration and potential energy $\phi$(r), which are values used in physics, can be represented as shown below when the coordinates of the origin are (x,y)=(0,0):

$$
\begin{aligned}
\mathtt{f(r)} \;&=\mathtt{-kr} \\
&=\mathtt{-k(xi+yj)} \tag{5}
\end{aligned}
$$

$$
\begin{aligned}
\mathtt{\phi(r)} \;&=\mathtt{Kr^2} \\
&=\mathtt{K(x^2+y^2)} \tag{6}
\end{aligned}
$$

where r, i, and j are vector values.

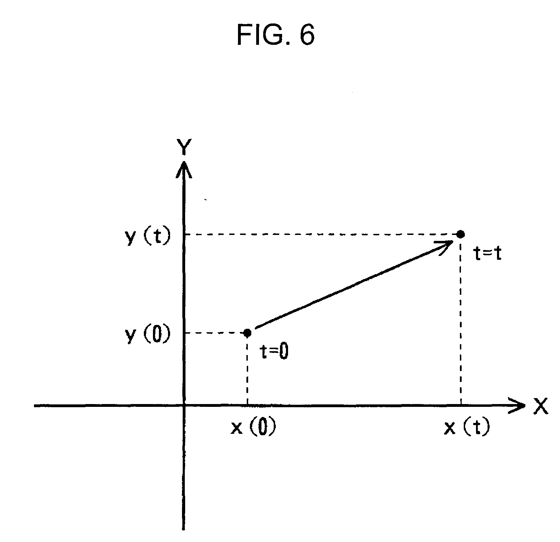

Accordingly, as illustrated in Fig. 6, if the origin of the coordinates is set to (x(0), y(0)) for time t=0, the force f(r) of a two-dimensional harmonic vibration and potential energy $\phi$(r) at time t=t is determined as shown below:

$$
\begin{aligned}
\mathtt{f(r)} \;&=\mathtt{-kr} \\
&=\mathtt{-(k1(x(t)-x(0))+k2(y(t)-y(0))} \\
&=\mathtt{-k1(x(t)-x(0))-\ k2(y(t)-y(0))} \tag{7}
\end{aligned}
$$

$$
\begin{aligned}
\mathtt{\phi(r)} \;&=\mathtt{kr^2} \\
&=\mathtt{k1(x(t)-x(0))^2+k2(y(t)-y(0))^2)} \tag{8}
\end{aligned}
$$

where x(t) and y(t) represent the coordinates at time t, x(0) and y(0) represent the coordinates (initial values or reference coordinates) at time t=0, and k1 and k2 are constants.

In this example, the electromyographic activity v(t) is obtained by Formula (9), which is derived from Formulas (7) and (8), below:

$$
\begin{aligned}
\mathtt{v(t)} \;&=\mathtt{f(r)\times\phi(r)} \\
&=\mathtt{(-k1(x(t)-x(0))-k2(y(t)-y(0))} \\
&\quad\mathtt{\times((k1(x(t)-x(0))^2+k2(y(t)-y(0))^2)} \tag{9}
\end{aligned}
$$

Here, the force f(r) of a two-dimensional harmonic vibration and the potential energy $\phi$(r) are multiplied so that v(t) has both a positive value and a negative value and thus the multiplication has no meaning from the point view of physics. In other words, when the electromyographic activity of the face is directly measured, a signal including both a positive value and a negative value is obtained. In order to obtain a similar signal, the force f(r) of a two-dimensional harmonic vibration and the potential energy $\phi(r)$ are multiplied. Formula (9) is used to compute the direction and amount of displacement (variation) of the positions (or distance) of the measuring points set on the user's face.

[3] Other Descriptions

As described above, the bio-information sensors may be any type of sensor capable of measuring the facial expression, voice, body movement, respiration, pulse rate, perspiration, skin surface temperature, micro-vibration (MV), electrocardiographic activity, electromyographic activity, blood oxygen level, skin resistance, and blinking of a user (subject). As the user's psychological state, emotion and mood may be inferred from the measurements.

Moreover, when changing an image signal and/or a sound signal based on the user's psychological state and when its intensity is being inferred from the measurements, the reproduction speed, volume, color, and/or content of images and/or sound may be modified. The image signals and sound signals modified based on the measured bio-information may be recorded.

As a recording medium, the hard disk drive 24, an optical disk, a magneto-optical disk, a magnetic tape, a hard disk, a semiconductor memory, or an integrated chip (IC) card may be used. The optical disk may be a compact disk (CD), a CD-Recordable (CD-R), a CD-ReWritable (CD-RW), a mini disc, a DVD-Recordable (DVD±R), a DVD-Re-Writable (DVD±RW), a DVD random access memory (DVD-RAM), or a Blu-ray Disc. As described above, image signals and sound signals can be modified based on bio-information. A setting may be provided for selecting whether or not to accept the modification.

As described above, the image and/or sound reproduction conditions are controlled based on computed values of arousal and valence. Instead of controlling images and/or sound reproduction based on the values of arousal and valence, the environment of the user, such as the user's house, office, and relationship with other people, can be assessed, or usability of products can be assessed. Furthermore, the results of computing arousal and valence can be displayed as graphs and numerals.

It should be understood by those skilled in the art that various modifications, combinations, sub-combinations and alterations may occur depending on design requirements and other factors insofar as they are within the scope of the appended claims or the equivalents thereof.

**Claims**

1. A bio-information processing apparatus, comprising:

   a plurality of bio-information sensors for obtaining a plurality of measured bio-information values of a subject and outputting the plurality of measured bio-information values as a plurality of biological signals; and
   a circuit for estimating the psychological state and intensity of the psychological state of the subject from the plurality of biological signals and from one of initial bio-information values and reference bio-information values.

2. The bio-information processing apparatus according to Claim 1, wherein at least one of the plurality of measured bio-information values is one of respiration rate, pulse rate, and electromyographic activity of the subject.

3. The bio-information processing apparatus according to Claim 2, wherein the psychological state of the subject is at least one of emotion, mood, arousal, and valence.

4. The bio-information processing apparatus according to Claim 3, wherein at least one of the bio-information sensors is a sensor in contact with the subject.

5. The bio-information processing apparatus according to Claim 3 or 4, wherein at least one of the bio-information sensors is a sensor not in contact with the subject.

6. A video/sound reproduction apparatus, comprising:

   reproduction means for reproducing at least one of an image signal and a sound signal;
   a plurality of bio-information sensors for obtaining a plurality of measured bio-information values of a subject and outputting the plurality of measured bio-information values as a plurality of biological signals;
   means for estimating the psychological state and intensity of the psychological state of the subject from the plurality of biological signals and from one of initial bio-information values and reference bio-information values; and

modification means for modifying at least one of the image signal and the sound signal reproduced by the reproduction means in accordance with the results estimated by the circuit.

7. The video/sound reproduction apparatus according to Claim 6, wherein at least one of the bio-information sensors is a sensor in contact with the subject.

8. The video/sound reproduction apparatus according to Claim 6 or 7, wherein at least one of the bio-information sensors is a sensor not in contact with the subject.

9. The video/sound reproduction apparatus according to any one of Claims 6 to 8, wherein at least one of the bio-information sensors is capable of measuring at least one of facial expression, voice, body movement, respiration, pulse rate, perspiration, skin surface temperature, micro-vibration, electrocardiographic activity, electromyographic activity, blood oxygen level, skin resistance, blinking, and eye movement of the subject.

10. The video/sound reproduction apparatus according to any one of Claims 6 to 8, wherein the psychological state of the subject is at least one of emotion, mood, arousal, and valence.

11. The video/sound reproduction apparatus according to Claim 10, wherein the arousal is determined based on fluctuation of at least one of heart rate, respiration rate, and pulse rate of the subject.

12. The video/sound reproduction apparatus according to Claim 10 or 11, wherein the valence is determined based on change in at least one of facial expression and electromyographic activity of the subject.

13. The video/sound reproduction apparatus according to any one of Claims 6 to 12, wherein the modification means modifies at least one of reproduction speed, volume, color, and content of at least one of the image signal and the sound signal.

14. The video/sound reproduction apparatus according to any one of Claims 6 to 13, further comprising recording means for recording at least one of bio-information, and a sound signal and an image signal modified based on the bio-information.

15. The video/sound reproduction apparatus according to Claim 14, wherein the recoding means is one of an optical disk, a magneto-optical disk, a magnetic tape, a hard disk, a semiconductor memory, and an integrated circuit card.

16. The video/sound reproduction apparatus according to Claim 15, wherein the optical disk is one of a compact disk, a compact disk-Recordable, a compact disk-ReWritable, a mini disc, a digital versatile disk-Recordable, a digital versatile disk-ReWritable, a digital versatile disk random access memory, and a Blu-ray Disc.

17. The video/sound reproduction apparatus according to any one of Claims 6 to 16, wherein a user is capable of selecting whether to approve or forbid the modification of at least one of an image signal and a sound signal based on the bio-information.

# FIG. 1

THERMOGRAPHY `11`

VIDEO CAMERA `12`

RESPIRATION SENSOR `13`

PULSE BEAT SENSOR `14`

ELETRO MYOGRAPHIC SENSOR `15`

BIO INFORMATION ANALYSIS CIRCUIT `16`

TRANSMISSION AND RECEPTION `31`

COMMUNICATION `32`

`40`

CPU `21`

`29`

ROM `22`

RAM `23`

HDD `24`

KEY BOARD/ MOUSE `25`

VIDEO/ SOUND CONTROL `26`

DISPLAY CONTROL `27`

SOUND PROCESSING `28`

`39`

DVD PLAYER `36`

DISPLAY `37`

`38`

`20`

EP 1 609 418 A1

## FIG. 2

EP 1 609 418 A1

PULSE WAVE

ELETROMYOGRAPHIC ACTIVITY
(CORRUGATOR MUSCLE)

RESPIRATION

ELETROMYOGRAPHIC ACTIVITY
(CHEEK BONE MUSCLE)

SECOND

SECOND

# FIG. 3

ORBICULARIS
MUSCLE

CORRUGATOR
MUSCLE

CHEEK BONE
MUSCLE

FIG. 4

100

OBTAIN BIO
INFORMATION
101

COMPUTE
AROUSAL
AND VALENCE
102

| AROUSAL > 0 | AROUSAL > 0 | AROUSAL < 0 | AROUSAL < 0 |
| VALENCE > 0 | VALENCE < 0 | VALENCE > 0 | VALENCE < 0 |
103

KEEP AS IS    DECREASE    INCREASE

111    112    113

EP 1 609 418 A1

# FIG. 5

FIG. 6

## EUROPEAN SEARCH REPORT

Application Number

EP 05 25 3655

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2003/060728 A1 (MANDIGO LONNIE D) 27 March 2003 (2003-03-27) * paragraphs [0004] - [0006], [0015] - [0017], [0025]; figures 1,4 * | 1-4,6,7, 9-17 | A61B5/16 |
| Y | ----- | 8 | |
| X | US 2003/012253 A1 (PAVLIDIS IOANNIS) 16 January 2003 (2003-01-16) * abstract; figures 1,3,5,6 * * paragraphs [0032], [0033], [0080], [0127], [0128] * | 1,5 | |
| Y | ----- | 8 | |
| X | US 2003/009078 A1 (FEDOROVSKAYA ELENA A ET AL) 9 January 2003 (2003-01-09) * paragraphs [0002], [0006], [0056] - [0067], [0070]; figures 1,2 * ----- | 1,3,6, 10,17 | |
| X | US 2001/028309 A1 (TORCH WILLIAM C) 11 October 2001 (2001-10-11) * abstract; figures 1,2,11A * * paragraphs [0019], [0020] * ----- | 1,5,6,8, 9 | TECHNICAL FIELDS SEARCHED (Int.Cl.7)<br><br>A61B |
| X | WO 02/100267 A (COMPUMEDICS LIMITED; BURTON, DAVID; ZILBERG, EUGENE) 19 December 2002 (2002-12-19) * abstract; figures 15-17 * * page 22, line 30 - page 23, line 9 * * page 33, line 16 - page 34, line 11 * | 1,2 | |
| A | ----- | 9,11,12 | |
| A | US 2003/069516 A1 (BECKER CRAIG HENRY ET AL) 10 April 2003 (2003-04-10) * abstract; figure 1 * * paragraphs [0005], [0016] * ----- | 3,9,10, 12 | |
| | -/-- | | |

The present search report has been drawn up for all claims

| Place of search | Date of completion of the search | Examiner |
|---|---|---|
| Berlin | 26 August 2005 | Jonsson, P.O. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | MORISHIMA S: "Modeling of facial expression and emotion for human communication system" DISPLAYS, ELSEVIER SCIENCE PUBLISHERS BV., BARKING, GB, vol. 17, no. 1, August 1996 (1996-08), pages 15-25, XP004032536 ISSN: 0141-9382 * paragraphs [0001], [0005], [0006]; figures 19-21,25 * ----- | 1-17 | |

TECHNICAL FIELDS SEARCHED (Int.Cl.7)

The present search report has been drawn up for all claims

| Place of search | Date of completion of the search | Examiner |
|---|---|---|
| Berlin | 26 August 2005 | Jonsson, P.O. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 05 25 3655

This annex lists the patent family members relating to the patent documents cited in the above-mentioned European search report.
The members are as contained in the European Patent Office EDP file on
The European Patent Office is in no way liable for these particulars which are merely given for the purpose of information.

26-08-2005

| Patent document cited in search report | | | Publication date | Patent family member(s) | | Publication date |
|---|---|---|---|---|---|---|
| US 2003060728 | A1 | | 27-03-2003 | NONE | | |
| US 2003012253 | A1 | | 16-01-2003 | EP | 1383419 A1 | 28-01-2004 |
| | | | | JP | 2004529702 T | 30-09-2004 |
| | | | | WO | 02085197 A1 | 31-10-2002 |
| | | | | EP | 1381308 A1 | 21-01-2004 |
| | | | | JP | 2004529703 T | 30-09-2004 |
| | | | | US | 2003016726 A1 | 23-01-2003 |
| | | | | WO | 02085198 A1 | 31-10-2002 |
| US 2003009078 | A1 | | 09-01-2003 | NONE | | |
| US 2001028309 | A1 | | 11-10-2001 | US | 6163281 A | 19-12-2000 |
| | | | | US | 5748113 A | 05-05-1998 |
| | | | | US | 6246344 B1 | 12-06-2001 |
| | | | | AU | 4583399 A | 10-01-2000 |
| | | | | WO | 9967757 A1 | 29-12-1999 |
| WO 02100267 | A | | 19-12-2002 | WO | 02100267 A1 | 19-12-2002 |
| | | | | CA | 2448806 A1 | 19-12-2002 |
| | | | | CN | 1538823 A | 20-10-2004 |
| | | | | EP | 1395176 A1 | 10-03-2004 |
| | | | | JP | 2005506115 T | 03-03-2005 |
| | | | | US | 2004193068 A1 | 30-09-2004 |
| US 2003069516 | A1 | | 10-04-2003 | NONE | | |

EPO FORM P0459